# EUROPEAN PATENT SPECIFICATION

(11) **EP 2 927 326 B1**
(45) Date of publication and mention of the grant of the patent: **07.11.2018**
(21) Application number: 14162563.2
(22) Date of filing: 31.03.2014
(51) Int. Cl.: C12Q 1/68

(54) **Method and tools for determining DNA fragmentation**
Verfahren und Werkzeuge zur Bestimmung von DNA-Fragmentierung
Procédé et outils pour la détermination de la fragmentation de l'ADN

(43) Date of publication of application: 07.10.2015
(73) Proprietor: FertiPro N.V., 8730 Beernem (BE)
(72) Inventor: Comhaire, Sven, 8620 Nieuwpoort (BE); Comhaire, Björn, 9880 Lotenhulle (BE); Eertmans, Frank, 8730 Beernem (BE); Bogaert, Veerle, 8730 Beernem (BE)
(74) Representative: Paemen, Liesbet R.J.

(56) References cited:
- EP-A1- 1 710 320
- FERNANDEZ J L ET AL: "Simple determination of human sperm DNA fragmentation with an improved sperm chromatin dispersion test", FERTILITY AND STERILITY, ELSEVIER SCIENCE INC, NEW YORK, NY, USA, vol. 84, no. 4, 1 October 2005 (2005-10-01), pages 833-842, XP027699690, ISSN: 0015-0282 [retrieved on 2005-10-01]
- A W Gustafson: "Cells, Tissues & Organs: Methods of Study", , 1 January 2006 (2006-01-01), pages 1-10, XP055131170, Retrieved from the Internet: URL:http://ocw.tufts.edu/data/15/342518.pd f [retrieved on 2014-07-23]
- JEKATARINA ERENPREISA ET AL: "Apoptotic cell nuclei favour aggregation and fluorescence quenching of DNA dyes", HISTOCHEMISTRY AND CELL BIOLOGY, vol. 108, no. 1, 1 July 1997 (1997-07-01), pages 67-75, XP055130593, ISSN: 0948-6143
- FERNANDEZ J L ET AL: "THE SPERM CHROMATIN DISPERSION TEST: A SIMPLE METHOD FOR THE DETERMINATION OF SPERM DNA FRAGMENTATION", JOURNAL OF ANDROLOGY, J.B. LIPPINCOTT CO., PHILADELPHIA, US, vol. 24, no. 1, 1 January 2003 (2003-01-01), pages 59-66, XP008052005, ISSN: 0196-3635
- Mary Johnson: "Detergents: Triton X-100, Tween-20, and More", Mater Methods, 21 January 2014 (2014-01-21), XP055131724, Retrieved from the Internet: URL:http://www.labome.com/method/Detergent s-Triton-X-100-Tween-20-and-More.html [retrieved on 2014-07-25]
- JEKATERINA ERENPREISA ET AL: "Toluidine blue test for sperm DNA integrity and elaboration of image cytometry algorithm", CYTOMETRY, vol. 52A, no. 1, 20 February 2003 (2003-02-20), pages 19-27, XP55067798, ISSN: 0196-4763, DOI: 10.1002/cyto.a.10015
- ALI TALEBI ET AL: "Sperm chromatin condensation, DNA integrity, and apoptosis in men with spinal cord injury", THE JOURNAL OF SPINAL CORD MEDICINE, vol. 36, no. 2, 1 March 2013 (2013-03-01), pages 140-146, XP055130411, ISSN: 1079-0268, DOI: 10.1179/2045772312Y.0000000055

## Description

### FIELD OF THE INVENTION

Provided herein are methods and tools for determining the fragmentation of nuclear DNA in cells such as sperm cells.

### BACKGROUND OF THE INVENTION

DNA fragmentation is the breaking of DNA strands into fragments. It can be induced *in vitro* in the context of DNA analysis but is also observed *in vivo* during cell apoptosis. Nuclear DNA damage in sperm is one of the major factors affecting embryo quality, implantation and development and the determination of DNA fragmentation is thus of key importance in fertility screening and artificial reproduction treatments.

Different methods are known in the art for the detection of DNA fragmentation. A widely used test for assessing sperm quality is the Sperm Chromatin Structure Assay (SCSA). Another type of assay, referred to as the TUNEL (terminal deoxynucleotidyl transferase end labeling) is based on the labeling of the terminal end of nucleic acids with fluorescent-dUTP by a transferase enzyme. However, the SCSA and TUNEL assays typically require flow cytometry detection, which often is not available for routine analysis. Other assays, such as the Sperm Chromatin Dispersion (SCD) test, use detection via imaging techniques. In the SCD test, unfixed sperm cells are incubated in an acid unwinding solution to obtain single-stranded DNA, and are then lysed and stained. The sperm cells with intact DNA can be identified as displaying big halo's of DNA loops, whereas the sperm cells with fragmented DNA show no or only small halos. EP1710320 describes a SCD assay for the detection of DNA fragmentation in sperm cells, which involves treating the cells with a denaturing solution, a lysis step using non-ionic detergents and analysis of the integrity of the DNA. WO2013064159 describes a method for assessing fragmentation of nuclear DNA in sperm sample by means of Raman micro-spectroscopy.

There remains a need for an improved simple and reliable test for determining DNA fragmentation in cells, more particularly human sperm cells.

### SUMMARY OF THE INVENTION

Disclosed herein are methods and tools which allow a simple, reliable determination of DNA fragmentation in cells, more particularly human sperm cells. The advantage of the claimed methods and tools is a more reliable detection of DNA fragmentation, while not requiring an extensive laboratory setup.

The methods envisaged herein are useful to detect the presence of intact and/or damaged nuclear DNA in sperm.

More particularly, provided herein are methods for determining DNA fragmentation of sperm cells in a semen sample, comprising exposing the cells to a lysis buffer comprising an ionic detergent. In the methods provided herein the sample is thereafter exposed to a denaturing solution and stained. In particular embodiments, the methods comprise exposing the cells to a lysis buffer comprising an ionic detergent, thereafter exposing the cells to a denaturing solution; thereafter staining the cells with a staining solution comprising a metachromatic staining reagent; and finally determining the extent of DNA fragmentation based on the intensity of the staining of the cells.

In particular embodiments of the present methods, the ionic reagent is sarkosyl.

In certain embodiments, the denaturing reagent is HCI.

In particular embodiments, the staining reagent is toluidine blue.

In certain embodiments, the sample is provided on an agarose support film.

In particular embodiments, the sample is provided in an agarose solution.

In certain embodiments, the present method is a method for fertility screening.

Further provided herein are kits for performing the method for determining DNA fragmentation as described herein. The kits provided herein typically comprise a lysis buffer comprising an ionic reagent in a concentration between 0.2% w:v and 0.8% w/v, dithiothreitol (DTT) in a concentration between 0.1M and 0.25M; Tris between 0.1M and 0.3M and sodium chloride in a concentration between 0.1M and 0.2M, a denaturing solution and a staining solution. In particular embodiments, the kits comprise a lysis buffer comprising an ionic reagent, a denaturing solution comprising an acid reagent, and (c) a staining solution comprising a metachromatic staining reagent.

In certain embodiments, the kits may also comprise a low-melting agarose solution.

In particular embodiments, the kits may comprise an agarose support film.

In certain embodiments of the kits as described above, the staining reagent is Toluidine Blue.

In particular embodiments of the kits as described above, the ionic reagent is sarkosyl.

### DETAILED DESCRIPTION

While potentially serving as a guide for understanding, any reference signs in the claims shall not be construed as limiting the scope thereof.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" when referring to recited components, elements or method steps also include embodiments which "consist of" said recited components, elements or method steps.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments described herein are capable of operation in other sequences than described or illustrated herein.

The values as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to ensure one or more of the technical effects envisaged herein. It is to be understood that each value as used herein is itself also specifically, and preferably, disclosed.

The recitation of numerical ranges by endpoints includes all numbers and fractions subsumed within the respective ranges, as well as the recited endpoints.

Unless otherwise defined, all terms used in disclosing the concepts described herein, including technical and scientific terms, have the meaning as commonly understood by one of ordinary skill in the art. By means of further guidance, definitions for the terms used in the description are included to better appreciate the teaching of the present disclosure. The terms or definitions used herein are provided solely to aid in the understanding of the teachings provided herein.

Provided herein are methods and tools for the determination of fragmentation of nuclear DNA (nDNA) in cells, more particularly sperm cells.

The methods are characterized in that they involve a lysis step using a lysis solution comprising an ionic detergent, a denaturation step and a staining step, followed by detection of color intensity of the cells. More particularly envisaged herein are methods comprising the steps of (a), exposing the cells to a lysis solution comprising an ionic detergent and a reducing agent, (b) denaturation of the cells using an acidic denaturing solution, (c) staining the cells and (d) determining the degree of staining as a measure for DNA fragmentation. The different steps of the methods and preferred embodiments for carrying them out are detailed below. It will be understood by the skilled person that while for all steps the different options and variations described herein are possible to obtain a satisfactory result, optimal results have been obtained by combining at least two, most particularly at least three or more of the preferred embodiments for each of the steps detailed herein.

The methods envisaged herein thus involve a first step of lysis of the cells by contacting the cells with an ionic detergent. In particular embodiments the method involves only one lysis step, i.e. no other solutions involving ionic or other cell-membrane permeating agents (such as non-ionic protein denaturing agents) are used. In particular embodiments, the lysis step consists of bringing the cells into contact with a lysis buffer, which is a buffer comprising an ionic agent. Indeed it has been found that, in contrast to established concepts, ionic agents can successfully be used in the determination of DNA fragmentation of cells such as sperm cells, without resulting in excessive damage of the cells. More particularly it has been found that such a treatment can be used without loss of the sperm tail. Ionic detergents are known in the art and include both anionic and cationic detergents such as, but not limited to sodium dodecyl sulphate (SDS), alkyl benzene sulphonate, glycolic acid hydrated salt, cholic acid, Sodium lauroyl sarcosine (or sarkosyl), etc. In preferred embodiments, the ionic detergent is sarkosyl. It is envisaged that the ionic detergent is present in a concentration in the lysis buffer between 0.2% w/v and 0.8% w/v. In particular embodiments, the ionic detergent is present in a concentration of about 0.5% w/v. In preferred embodiments, the ionic detergent is a sarkosyl present in a concentration of about 0.5% w/v.

The lysis buffer for use in the methods envisaged herein will typically further include a reducing agent such as DTT or beta-mercaptoethanol. Further the lysis buffer will typically contain a buffering agent such as Tris, Hepes, Mops, Pipes, and one or more salts, such as sodium chloride.

In particular embodiments, the lysis buffer, in addition to the ionic detergent will comprise: dithiothreitol (DTT) between 0.001 M and 2M, preferably between 0.01 M and 0.8M; 2-amino-2 (hydroxymethyl)-1,3-propanediol (Tris) between 0.001 M and 2M, preferably between 0.01 M and 0.4M; sodium chloride (NaCI) between 0.1M and 3M, preferably between 0.1M and 1M. In preferred embodiments, the lysis buffer, is composed of an ionic detergent such as sarkosyl as detailed above and further comprises DTT between 0.1 M and 0.25M, Tris between 0.1M and 0.3M, and NaCl between 0.1M and 0.2M.

Further optional components of the lysis buffer are components such as Triton (e.g. t-octylphenoxypolyethoxyethanol or Triton X-100) or other non-ionic or neutral detergents (preferably between 0.1% and 3%, more particularly between 0.5%-1.5%). Suitable non-ionic detergents may include, but are not limited to t-octylphenoxypolyethoxyethanol (e.g. Triton X-100), bigCHAP (N,N-bis[3-(D-Gluconamido)propyl]cholamide), Polyethylene glycol dodecyl ethers (e.g. Brij® 35 P), N-decanoyl-N-methylglucamine, digitonin, dodecanoyl-N-methylglucamide, heptanoyl-N-methylglucamide, branched octylphenoxy poly (ethyleneoxy) ethanol (e.g. Igepal CA-630), N-Nonanoyl-N-methylglucamine, octylphenoxypolyethoxyethanol (Nonidet P 40), N-Octanoyl-N-methylglucamine, sorbitan monolaurate (Span® 20), and Polysorbates (e.g. polyoxyethylene sorbitan monolaurate or Tween 20). In particular embodiments, the lysis buffer does not comprise any additional neutral components.

The lysis solution typically has a pH which is adjusted to between 6.5 and 8.0, more preferably between 6.75 and 6.85, for example about 6.8.

The contact time between the sample and the lysis solution in the lysis step of the methods envisaged herein may vary but is typically between 5-25 minutes. For a lysis solution containing 0.5% w/v sarkosyl, optimal results have been obtained within 10 minutes.

The methods envisaged herein further envisage exposing the cells to a denaturing solution, more particularly by contacting the cells with the denaturing solution. The denaturing solution preferably comprises an acid, such as but not limited to one or more of a hydrochloric acid, an acetic acid, a nitric acid etc. In particular embodiments, the acid is hydrochloric acid. Typically, the concentration of hydrochloric acid in the denaturing solution varies between 0.01M and 0.5M, more particularly between 0.01M and 1.5M, most particularly, the concentration is around 0.08M. In preferred embodiments, the denaturing solution comprises hydrochloric acid in a concentration between 0.01 and 0.1M.

The time required to ensure denaturation of the sample may vary, but will typically vary between 1 and 15 minutes, more particularly between 5 and 7 minutes. Satisfactory results are obtained by incubating a sample with a hydrochloric acid containing buffer of between 0.01 and 0.1M for 5 minutes. Preferably, denaturation is performed at a temperature between 1°C and 37°C, preferably between 18°C and 25°C, for example about 20°C.

The methods envisaged herein further comprise a staining step. Typically this step is performed after the lysis and denaturation steps. It has been found that when performing the methods comprising the lysis and denaturation step as described herein, in combination with a chromatin staining step, this allows sensitive and quantitative detection of nuclear DNA fragmentation in the cells. In particular embodiments, the staining step includes exposing the cells to a staining solution comprising a metachromatic staining reagent. Such metachromatic dyes are mainly of the thiazine group, thionine, toluidine blue, azure A, azure B, methyl violet, safranin and acridine orange. In preferred embodiments, the staining solution comprises toluidine blue (TB). It has been found that staining with TB in the methods described herein allows a quantification of DNA fragmentation based on the intensity of the blue staining. More particularly samples with no DNA fragmentation are not colored or are stained only faintly blue, samples with medium DNA fragmentation are stained blue, while samples with a high amount of DNA fragmentation in the cells are stained dark blue.

The concentration of the staining reagent, such as of TB, in the staining solution is typically between 0.01 and 0.1%w/v, such as about 0.05%w/v. The staining reagent is typically provided in a buffer solution such as phosphate buffers, citrate phosphate buffers, sodium acetate buffers, or potassium hydrogen phthalate/ sodium hydroxide buffers systems.

In particular embodiments, the buffer is a two-component buffer, such as Mcllvain buffer (comprising Na₂HPO₄ and citric acid). The concentration of the buffer may be between 25-100%.The inventors have found that staining solutions comprising about 50% Mcllvain buffer provide the best results.

In particular embodiments the pH of the staining solution has a pH between 2 and 6, such as at a pH of about 5.5. In preferred embodiments, the staining solution comprises TB in a buffer with a pH of about 5.5. The temperature during staining is not critical. In particular embodiments, staining is performed at a temperature between 1°C and 37°C.

The time required to ensure staining of the cells will depend on different factors, such as the choice and concentration of the staining reagent. In particular embodiments, the sample is exposed to or contacted with the staining solution for a time period between 1 and 30 minutes, more particularly between 1 and 10 minutes. Satisfactory results have been obtained by bringing the cells in contact with an 0.05% TB staining solution for between 3 and 8 minutes.

Although it is envisaged that the sequence of the steps detailed above can be made in any order, particularly beneficial results have been obtained by first treating the cells with the lysis solution and thereafter with the denaturing solution, followed by staining. The methods will typically comprise additional steps prior to and after or in between the steps recited above. For instance, the method will typically comprise a sample preparation step, prior to the lysis and denaturation steps recited above. Furthermore, the method may comprise a dehydratation step, washing steps, and/or additional staining steps, as will be detailed herein below.

The preparation of the sample prior to carrying out the lysis and denaturation steps typically involves determining the concentration of cells, such as semen, in the sample. Representative detection using the methods envisaged herein is most likely to be ensured when using samples comprising cells in a concentration between 1 and 200 million cells/ml, preferably between 1 and 40 million cells/ml. Methods for determining the concentration of cells such as sperm cells are known in the art. It will be understood to the skilled person that in particular embodiments, the methods may comprise adjusting the concentration of the cells in the sample by dilution with a buffer or concentration. Preferably the concentration of cells in the sample is adjusted to about 10 million cells/ml.

In order to allow optical detection of DNA fragmentation of cells in a sample as envisaged in the methods described herein, it is of interest to provide the cells on a support, such as a glass slide or a (glass bottomed) well-plate.

In particular embodiments, the cells may be embedded in an inert medium. This may be obtained by suspending the cells a solution of the inert medium, which is subsequently deposited on a solid support. After drying of the solution, the cells are embedded and immobilized in the inert medium. In particular embodiments, the support may be precoated with a film of the inert medium to increase the compatibility of the solution comprising the cells with the support. It is also possible to use a film of the inert medium as such as the solid support.

In preferred embodiments, the inert medium is agarose. The cell-containing sample can be provided on to the support in an agarose-containing suspension by mixing the sample with a low-melting point agarose solution. Agarose which has been modified (e.g. by introduction of hydroxyethyl groups) to ensure a lower melting point is commercially available. Typically, the agarose is heated (in a warm water bath at temperature between 90°C and 100°C or in a microwave oven) and then briefly incubated in a water bath at 37°C prior to use. A sample of cells is mixed with a volume of agarose and thereafter provided onto the support. Examples of suitable concentrations of agarose solutions are known in the art (typically between 0.5% and 2% in distilled water or in a buffer such as TBE or TAE). The agarose and sample are typically mixed in a relative concentration of 70%-30%, respectively. In further embodiments, the support is or comprises an agarose (support) film, such as a Gelbond® film. This ensures an optimal compatibility between the agarose solution comprising the cells and the support. For easy handling, the agarose support film is placed on a solid surface (e.g. a glass slide or plastic plate) with the hydrophilic side upwards, taking care to avoid air bubbles so as to ensure a smooth horizontal surface. The cell mixture is mixed with agarose and then transferred onto the agarose support film. The mixture is then covered with a cover glass and placed at 4°C between 2-30 minutes to allow gelling of the agarose. The sample is then ready for treatment using the envisaged method, i.e. with the lysis and denaturation steps as described hereinabove. Conveniently, the cover glass is removed from the agarose support film while the film is still supported by the solid surface. Thereafter, the agarose support film can be handled without the solid surface, e.g. by placement in a cassette. The lysis and denaturation steps as performed on a sample prepared as described above, can advantageously be performed in 10 and 5 minutes, respectively.

As detailed above, the method may involve a washing step after the lysis step and/or after the denaturation step and/or after the staining step. Typically this involves bringing the cells once or several times in contact with an aqueous solution such as distilled water, physiological saline or a buffer, for 0.5-30 minutes. In preferred embodiments, the sample exposed to the denaturing solution directly after the lysis solution, with no intermediate washing step. In further particular embodiments, the sample is washed after the denaturation step and after the staining step. Satisfactory results have been obtained by washing the sample one or more times for 5 minutes with ultrapure water after the denaturation step. After the staining step the duration of the washing step is typically limited to less than 1 minute for example about 10 seconds, to avoid loss of staining intensity over time.

The methods envisaged herein typically involve a dehydration step prior to the staining step. This is typically ensured by exposing the sample to different solutions containing increasing concentrations of alcohol and/or another solvent. For instance, successive incubations with solutions of 70%, 90%, and 100% ethanol or acetone (w/v) can be used. Satisfactory results are obtained when the sample is brought into contact with each of these solutions for between 30 seconds and 30 minutes, such as for 2 minutes. The dehydration step is typically followed by a step wherein the sample is allowed to dry. The sample can then be exposed to the DNA staining solution as described above.

In particular embodiments the methods may further comprise one or more additional staining steps other than the DNA staining step which facilitates identification of the cells or detection of other properties of the cells. The additional staining can be of the same or a different type of staining as the chromatin stain used in the staining step described above. Suitable examples of staining methods are known in the art.

After the DNA staining step (and optionally further staining steps), the extent of DNA fragmentation in the cells is assessed. More particularly, the result of the DNA staining is assessed as a measure to determine DNA fragmentation in the cells. The method of detection typically depends on the nature of the stain used. Typically, for the metachromatic visual stains envisaged in particular embodiments of the methods disclosed herein, detection is performed using a clear field microscope. The intensity of the staining is typically determined for a representative number of cells in the sample, such as between 100-1000 cells, such as 500 cells. In particular embodiments, the staining reagent is a metachromatic reagent and staining intensities can be distinguished corresponding to different concentrations of fragmented DNA, allowing the discrimination of cells into categories based on the intensity of the staining. This assessment can be performed visually or by applying digital image analysis software on images obtained by cameras. In particular embodiments, the discrimination entails differentiating cells as either "no or limited stain", "stain" and "dark stain". Applicable to a preferred staining reagent such as TB the categories will correspond to "no or very clear/light blue stain", "blue stain" and "dark blue stain". The categories can be directly translated into a corresponding measure of DNA fragmentation.

As detailed above, the methods envisaged herein are particularly advantageous for the determination of nuclear DNA fragmentation in reproductive cells such as sperm cells. Thus, provided herein are methods and tools for determining DNA fragmentation in semen samples according to the steps described herein. These methods are used in the context of fertility screening and fertility treatment methods. Thus, the application envisages methods for determining the fertility of a male comprising analyzing the level of DNA fragmentation in a semen sample of said male by way of performing the steps described herein whereby high levels of DNA fragmentation are indicative of infertility of said male. Further the methods envisaged herein include methods for determining a fertility treatment for a couple in need thereof, which method comprises determining the level of DNA fragmentation in a semen sample of the male of said couple and selecting an appropriate fertility treatment based on the result of said determination.

The application further provides tools for carrying out the methods described herein, such as combinations of reagents or kits comprising reagents suitable for carrying out the envisaged methods. Kits are envisaged comprising a lysis solution comprising an ionic reagent in a concentration between 0.2% w:v and 0.8% w/v, dithiothreitol (DTT) in a concentration between 0.1M and 0.25M; Tris between 0.1M and 0.3M and sodium chloride in a concentration between 0.1M and 0.2M, and a denaturing solution as described herein above. In particular embodiments, the kits comprise combinations of the preferred embodiments of the lysis and denaturing solutions described herein above. Further the envisaged kits may include a staining solution as described above, such as the preferred TB staining solution. In yet further embodiments, the kit may comprise an agarose solution and/or a support, such as the agarose support film described herein. Preferably each of these reagents is provided in a separate container, such as a glass or plastic bottle or Eppendorf tube. Optional additional constituents of the kit are buffers. In particular embodiments, the kit as envisaged herein comprises:
- Lysis solution (comprising an ionic detergent such as sarkosyl in 0.5% w/v concentration); a reducing agent (such as DTT in a concentration of 0.2M), salt (such as NaCl at a concentration of about 170 mM) and a buffering agent (such as Tris at a concentration of 0.2M)
- Denaturing solution (such as HCI at a concentration of 0.08M)
- Staining (stock) solution (such as TB in a concentration between 0.1% and 5% w/v, for example about 1% w/v, in buffer). The staining solution provided in the kit may be a stock solution which comprises the staining reagent in a higher concentration than required for method described above. This may increase the stability and shelf life of the staining solution. The staining stock solution may then be diluted, for example using the dilution buffer described below, to obtain a staining solution having the desired concentration.

And one or more optional components such as:
- A low melting agarose solution.
- A support film, more particularly an agarose support film.
- A dilution buffer (such as 50% w/v Mcllvain: 28.6mL van 0.2M Na₂HPO₄ (0.2M) + 21.9mL 0.1M citric acid + 50.5mL H₂O (end pH= 5.5). The dilution buffer may be used for diluting the staining solution to the desired concentration prior to use.One or more containers for holding the sample (embedded in an inert medium), for example one or more (biopsy) cassettes.
- One or more reservoirs for use during washing steps.

In particular embodiments the kit as envisaged herein is limited to the above-recited constituting elements. The kit may however further comprise instructions for use of the kit according to the methods described herein.

### EXAMPLES

The following examples are provided for the purpose of illustrating the present invention and by no means are meant and in no way should be interpreted to limit the scope of the present invention.

### Reagents

- Low melting agarose (0.7% in 1x TBE)
- Lysis buffer:
   ∘ Sarkosyl (ionic detergent): 0.5% w/v
   ∘ DTT (reducing agent): 0.2M
   ∘ NaCl: 170mM
   ∘ Tris (buffering agent): 200mM
- Denaturing solution: HCI (0.08M)
- Stain: Toluidine blue (0.05%) in 50% Mcllvain, pH 5.5 (100% Mcllvain: 28.6mL 0.2M Na₂HPO₄ + 21.9mL 0.1M citric acid)

### Staining of a semen sample

Different samples of human semen were each analysed according to the protocol described below.
1. The semen sample was allowed to liquefy at room temperature.
2. The sample was then centrifuged at 500g for 10 minutes.
3. The pellet was dilute with PBS to a concentration of approximately 20million cells/mL.
4. An Eppendorf tube containing 50µl of low-melting agarose was placed for 5 minutes in a warm water bath at a temperature between 90°C-100°C.
5. The agarose was transferred to a warm water bath at 37°C and incubated for 5 minutes.
6. A piece of GelBond film was placed with the hydrophilic side upwards on top of a microscope glass.
7. 25uL of sperm solution was transferred to the agarose and mixed by pipetting up and down.
8. 20uL of this mix was transferred to the GelBond and covered with a cover glass.
9. The microscope glass with the GelBond and cover glass was placed at 4°C for 10 minutes.
10. The cover glass was removed by sliding it horizontally off the microscope glass.
11. The GelBond with sample was transferred to a cassette.
12. The Gelbond with sample was then incubated for 10 minutes in the lysis solution at room temperature.
13. The Gelbond with sample was then incubated for 5 minutes in a denaturing solution.
14. The Gelbond with sample was then washed in ultrapure water for 5 minutes.
15. The Gelbond with sample was successively incubated for 2 minutes in respectively 70%, 90% and 100% Ethanol.
16. The GelBond with sample was removed from the cassette and allowed to dry.
17. The staining solution was added to the sample on the Gelbond and incubated for 5 minutes.
18. The GelBond was then dipped 3 times in ultrapure water.
19. The GelBond was then allowed to air-dry.
20. 500 cells were counted.

The staining described above preserves the tails of the sperm cells, which facilitates the identification and counting of the cells. The degree of DNA fragmentation could be assessed based on the color intensity of the cells. This is found to be more reliable than the determination based on the differentiation between purple and blue cells according to prior art methods.

## Claims

1. A method for determining DNA fragmentation of sperm cells in a semen sample, comprising the following steps in the order provided:
(a) exposing the cells to a lysis buffer comprising an ionic detergent;
(b) exposing the cells to a denaturing solution;
(c) staining the cells with a staining solution comprising a metachromatic staining reagent; and
(d) determining the extent of DNA fragmentation based on the intensity of the staining of the cells;
wherein said method is **characterized in that** said lysis buffer does not destroy the sperm cell tails.

2. The method of claim 1, wherein said ionic reagent is sarkosyl.

3. The method of claim 1 or 2, wherein the denaturing reagent is HCI.

4. The method of any one of claims 1 to 3 wherein the staining reagent is toluidine blue.

5. The method of any one of claims 1 to 4, wherein said sample is provided on an agarose support film.

6. The method of any one of claims 1 to 5, wherein the sample is provided in an agarose solution.

7. The method of any one of claims 1 to 6, which is a method for fertility screening.

8. A kit for performing the method of any one of claims 1 to 7, comprising:
(a) a lysis buffer comprising an ionic reagent in a concentration between 0.2% w:v and 0.8% w/v, dithiothreitol (DTT) in a concentration between 0.1M and 0.25M; Tris between 0.1M and 0.3M and sodium chloride in a concentration between 0.1M and 0.2M;
(b) a denaturing solution comprising an acid reagent;
(c) a staining solution comprising a metachromatic staining reagent.

9. The kit of claim 8 further comprising a low-melting agarose solution.

10. The kit of claim 8 or 9, further comprising an agarose support film.

11. The kit of any one of claims 8 to 10, wherein the staining reagent is Toluidine Blue.

12. The kit of any one of claims 8 to 11 wherein said ionic reagent is sarkosyl.

## Patentansprüche

1. Verfahren zur Bestimmung der DNA-Fragmentierung von Spermien in einer Spermaprobe, umfassend die folgenden Schritte in der angegebenen Reihenfolge:
(a)Inkontaktbringen der Zellen mit einem ein ionisches Detergens umfassenden Lysepuffer;
(b)Inkontaktbringen der Zellen mit einer Denaturierungslösung;
(c)Färben der Zellen mit einer ein metachromatisches Färbereagens umfassenden Färbelösung; und
(d)Bestimmen des Ausmaßes der DNA-Fragmentierung anhand der Intensität der Färbung der Zellen;
wobei das Verfahren **dadurch gekennzeichnet ist, dass** der Lysepuffer die Spermiengeißeln nicht zerstört.

2. Verfahren nach Anspruch 1, wobei es sich bei dem ionischen Reagens um Sarkosyl handelt.

3. Verfahren nach Anspruch 1 oder 2, wobei es sich bei dem Denaturierungsreagens um HCl handelt.

4. Verfahren nach einem der Ansprüche 1 bis 3, wobei es sich bei dem Färbereagens um Toluidinblau handelt.

5. Verfahren nach einem der Ansprüche 1 bis 4, wobei die Probe auf einem Agarose-Trägerfilm bereitgestellt wird.

6. Verfahren nach einem der Ansprüche 1 bis 5, wobei die Probe in einer Agaroselösung bereitgestellt wird.

7. Verfahren nach einem der Ansprüche 1 bis 6, bei dem es sich um ein Verfahren für Fruchtbarkeit-Screening handelt.

8. Kit zur Durchführung des Verfahrens nach einem der Ansprüche 1 bis 7, umfassend:
(a)einen Lysepuffer, der ein ionisches Reagens in einer Konzentration zwischen 0,2% w:v und 0,8% w/v, Dithiothreitol (DTT) in einer Konzentration zwischen 0,1 M und 0,25 M; Tris zwischen 0,1 M und 0,3 M und Natriumchlorid in einer Konzentration zwischen 0,1 M und 0,2 M umfasst;
(b)eine Denaturierungslösung, die ein Säurereagens umfasst;
(c)eine Färbelösung, die ein metachromatisches Färbereagens umfasst.

9. Kit nach Anspruch 8, ferner umfassend eine niedrigschmelzende Agaroselösung.

10. Kit nach Anspruch 8 oder 9, ferner umfassend einen Agarose-Trägerfilm.

11. Kit nach einem der Ansprüche 8 bis 10, wobei es sich bei dem Färbereagens um Toluidine Blue handelt.

12. Kit nach einem der Ansprüche 8 bis 11, wobei es sich bei dem ionischen Reagens um Sarkosyl handelt.

## Revendications

1. Procédé de détermination de fragmentation d'ADN de spermatozoïdes dans un échantillon de sperme, comprenant les étapes suivantes dans l'ordre indiqué :
(a) exposition des cellules à un tampon de lyse comprenant un détergent ionique ;
(b) exposition des cellules à une solution de dénaturation ;
(c) coloration des cellules avec une solution de coloration comprenant un réactif de coloration métachromatique ; et
(d) détermination du degré de fragmentation d'ADN sur la base de l'intensité de la coloration des cellules ;
ledit procédé étant **caractérisé en ce que** ledit tampon de lyse ne détruit pas les flagelles des spermatozoïdes.

2. Procédé selon la revendication 1, dans lequel ledit réactif ionique est sarcosyle.

3. Procédé selon la revendication 1 ou 2, dans lequel le réactif de dénaturation est HCl.

4. Procédé selon l'une quelconque des revendications 1 à 3, dans lequel le réactif de coloration est le bleu de toluidine.

5. Procédé selon l'une quelconque des revendications 1 à 4, dans lequel ledit échantillon est disposé sur un film de support d'agarose.

6. Procédé selon l'une quelconque des revendications 1 à 5, dans lequel l'échantillon est disposé dans une solution d'agarose.

7. Procédé selon l'une quelconque des revendications 1 à 6, qui est un procédé pour un dépistage de fertilité.

8. Trousse pour conduire le procédé selon l'une quelconque des revendications 1 à 7, comprenant :
(a) un tampon de lyse comprenant un réactif ionique à une concentration comprise entre 0,2 % w/v et 0,8 % w/v, du dithiothréitol (DTT) à une concentration comprise entre 0,1 M et 0,25 M ; Tris entre 0,1 M et 0,3 M et du chlorure de sodium à une concentration comprise entre 0,1 M et 0,2 M ;
(b) une solution de dénaturation comprenant un réactif acide ;
(c) une solution de coloration comprenant un réactif de coloration métachromatique.

9. Trousse selon la revendication 8 comprenant en outre une solution d'agarose à point de fusion bas.

10. Trousse selon la revendication 8 ou 9, comprenant en outre un film de support d'agarose.

11. Trousse selon l'une quelconque des revendications 8 à 10, dans laquelle le réactif de coloration est le bleu de toluidine.

12. Trousse selon l'une quelconque des revendications 8 à 11, dans laquelle ledit réactif ionique est sarcosyle.
